# EUROPÄISCHE PATENTSCHRIFT

(11) **EP 2 694 118 B1**
(45) Veröffentlichungstag und Bekanntmachung des Hinweises auf die Patenterteilung: **12.11.2014**
(21) Anmeldenummer: 12713878.2
(22) Anmeldetag: 21.03.2012
(51) Int. Cl.: A61L 2/07

(54) **VERFAHREN ZUR DAMPFSTERILISATION VON MEDIZINISCHEN GÜTERN**
METHOD FOR STEAM STERILIZATION OF MEDICAL ARTICLES
STÉRILISATION D'INSTRUMENTS MÉDICAUX POUR L'INJECTION ET/OU L'INSTILLATION

(30) Priorität: 07.04.2011 DE 102011016377; 27.06.2011 DE 102011105840
(43) Veröffentlichungstag der Anmeldung: 12.02.2014
(73) Patentinhaber: Klosterfrau Berlin Gmbh, 12277 Berlin (DE)
(72) Erfinder: MEIER, Andreas, 13629 Berlin (DE); MIETHING, Holger, 12107 Berlin (DE)
(74) Vertreter: Strehlke, Ingo Kurt
(86) Internationale Anmeldenummer: PCT/EP2012/001250
(87) Internationale Veröffentlichungsnummer: WO 2012/136313

(56) Entgegenhaltungen:
- EP-A1- 1 484 069
- WO-A1-01/76646
- WO-A1-95/00180
- AU-B2- 526 378
- DE-A1- 10 114 946

## Beschreibung

Die vorliegende Erfindung betrifft das technische Gebiet der Sterilisation von medizinischen Behältnissen, wie medizinischen Instrumenten, Ampullen, Tuben oder dergleichen.

Insbesondere betrifft die vorliegende Erfindung ein Verfahren zur thermischen Sterilisation eines mit einem medizinischen Befüllungsgut befüllten Behältnisses nach Anspruch 1.

Unter dem Begriff Sterilisation versteht man im Rahmen der vorliegenden Erfindung die Abtötung bzw. irreversible Inaktivierung sämtlicher Mikroorganismen und Viren, einschließlich ihrer Ruhestadien, wie beispielsweise Endosporen, welche sich an bzw. in einem Objekt befinden. Da die vollständige Inaktivierung sämtlicher Mikroorganismen und Viren an bzw. in einem Objekt nicht mit absoluter Sicherheit gewährleistet werden kann, gilt ein Objekt bzw. eine Einheit eines Sterilisierguts im Allgemeinen als steril, wenn die Wahrscheinlichkeit einer Kontamination mit vermehrungsfähigen Mikroorganismen oder Viren höchstens 1 : 10⁶ beträgt. Dies bedeutet, dass unter einer Million Einheiten des Sterilisierguts höchstens eine Einheit mit einer koloniebildenden Einheit (KBE) eines Mikroorganismus kontaminiert ist bzw. dass der Restgehalt an vermehrungsfähigen Mikroorganismen und Viren je Einheit des Sterilisierguts höchstens 10⁻⁶ koloniebildende Einheiten (KBE) beträgt. Der Restgehalt von höchstens 10⁻⁶ koloniebildenden Einheiten je Einheit des Sterilisierguts wird auch als Sterility Assurance Level (SAL) bezeichnet.

Je nach Art des Sterilisierguts stehen unterschiedliche Sterilisierverfahren zur Verfügung, welche nach chemischen und physikalischen Sterilisierverfahren unterschieden werden.

Zu den chemischen Sterilisationsverfahren zählen beispielsweise Begasungen mit Formaldehyd oder Ethylenoxid, welche jedoch mit hohen Kosten sowie einem hohen verfahrenstechnischen Aufwand verbunden sind und aufgrund der Risiken durch den Einsatz gesundheitsgefährdender Stoffe nur für spezielle Anwendungen geeignet sind; auch aus regulatorischen Gründen ist der Einsatz dieser Verbindungen nicht immer möglich. So müssen beispielsweise Bereitschaftsverpackungen, in welche das zu sterilisierende Gut verpackt ist, für die Sterilisationsgase durchlässig sein. Weiterhin muss jedoch auch gewährleistet sein, dass die Gase nach Beendigung der Sterilisation wieder vollständig entfernt werden können, was sich in der Praxis äußerst schwierig gestaltet. Alternativ wird das Sterilisiergut unverpackt sterilisiert und muss anschließend unter sterilen Bedingungen, beispielsweise in Reinräumen, verpackt werden, was das Verfahren in der Durchführung aufwendiger und kostenintensiver macht.

Darüber hinaus sind diese Verfahren nicht zur Sterilisation von in verschlossenen Behältnissen gelagerten Gütern geeignet, da kein Kontakt zwischen dem Sterilisiergas und dem potentiellen Sterilisiergut hergestellt werden kann.

Bei der Wahl des Sterilisationsverfahrens werden daher in der Regel physikalische Verfahren bevorzugt, wenn die zu sterilisierenden Materialien bzw. Güter unter Sterilisationsbedingungen stabil sind. Gerade bei der Sterilisation verpackter bzw. in verschlossenen Behältnissen befindlicher Sterilisiergüter haben sich physikalische Verfahren bewährt.

Physikalische Sterilisationsverfahren werden in aktinische Verfahren, bei welchen die Mikroorganismen durch ionisierende Strahlungen abgetötet bzw. irreversibel inaktiviert werden, und thermische Verfahren, welche auf Hitzeeinwirkung beruhen, unterteilt.

Zu den aktinischen Verfahren zählen beispielsweise Bestrahlungen mit UV-, Gamma- oder Elektronenstrahlen, welche beispielsweise bei der industriellen Herstellung von medizinischen Einwegartikeln eingesetzt werden.

Die sterilisierende Wirkung der thermischen Verfahren beruht hingegen auf der hitzeinduzierten Denaturierung von Eiweißen, welche mit ihrer nativen Struktur auch ihre biologischen Fähigkeiten und Wirkungen verlieren, was in der Abtötung bzw. irreversiblen Inaktivierung der Mikroorganismen resultiert.

Zu den thermischen Sterilisierverfahren zählen insbesondere die Heißluftsterilisation und die Dampfsterilisation, wobei die Heißluftsterilisation aufgrund der schlechten Reproduzierbarkeit und der Empfindlichkeit gegenüber kleinsten Abweichungen vom idealen Verfahrensablauf - bedingt durch die schlechte Wärmeübertragung der Luft - nur für einige wenige Anwendungen geeignet ist.

Die Dampfsterilisation stellt hingegen den "Goldstandard" der Sterilisationsverfahren dar, bei welchem das Sterilisiergut standardgemäß mit 121 °C heißem und einen Überdruck von 2 bar absolut aufweisenden Wasserdampf 15 Minuten lang auf eine Temperatur von 121 °C erhitzt wird. Dieses synonym auch als "Sattdampfverfahren" bezeichnete Sterilisationsverfahren ist in hervorragender Weise reproduzierbar und automatisierbar und eignet sich auch zur Sterilisierung von in verschlossenen Behältnissen verpackten Gütern.

Zwar haben sich die Dampfsterilisationsverfahren in der alltäglichen Praxis bewährt und beruhen auf einer ausgereiften Technologie. Von Nachteil ist jedoch die oftmals lange Dauer der Sterilisation, welche eine ökonomisch sinnvolle Durchführung der Sterilisation erschwert und die Kosten für die eigentliche Verfahrensdurchführung und in der Folge auch für die sterilisierten Produkte erhöht. Dies gilt insbesondere vor dem Hintergrund, dass Dampfsterilisationen im Allgemeinen diskontinuierlich in Autoklaven durchgeführt werden. Auch können unerwünschte thermische Instabilitäten auftreten.

Eine Verkürzung der Sterilisationszeit, d. h. eine effektivere Sterilisation, würde zu einem höheren Durchsatz und somit zu einer wirtschaftlicheren Verfahrensführung führen. Hierzu müsste jedoch die Sterilisationstemperatur signifikant erhöht werden, was wiederum die Auswahl der Sterilisiergüter, welche dem Verfahren zugänglich sind, entscheidend einschränkt, da insbesondere Kunststoffe sehr empfindlich auf eine Temperaturerhöhung bei Einwirkung feuchter Hitze reagieren können.

Darüber hinaus haben die herkömmlichen Dampfsterilisationsverfahren den Nachteil, dass die durch den Dampf in die Sterilisationskammer eingetragene Energiemenge nicht effizient ausgenutzt wird. Bei Dampfsterilisationsverfahren bleibt daher immer ein Großteil der in die Sterilisationskammer eingetragenen Energie durch nichtkondensierten Dampf ungenutzt.

Die Anwendung des Sattdampfverfahrens wird in erster Linie durch die Feuchtigkeits- und Hitzeempfindlichkeit der zu sterilisierenden Materialien beschränkt. Die hohe Effektivität und Wirksamkeit des Sattdampfverfahrens liegt in der hohen Energiemenge begründet, welche durch den Dampf übertragen wird: So weist Wasser bei 121 °C und einem Druck von 2 bar eine Verdampfungsenthalpie von 2.199 kJ/kg auf, welche auch als latente Wärme bezeichnet wird. Bei der Kondensation des Dampfes an dem kühleren Sterilisiergut wird diese Wärmemenge auf das Sterilisiergut und sich gegebenenfalls auf diesem befindliche Mikroorganismen übertragen, wodurch einerseits Mikroorganismen direkt abgetötet bzw. irreversibel inaktiviert werden, andererseits verschlossene Behältnisse derart erwärmt werden können, dass auch ihr Inhalt sterilisiert werden kann - eine ausreichende Wärmeleitfähigkeit bzw. Wärmeübertragung im Inneren des Behältnisses vorausgesetzt.

Bei Sterilisiergütern, insbesondere verschlossenen Behältnissen, in deren Innern sich während oder insbesondere nach der Sterilisation, insbesondere aufgrund deren Befüllung, Drücke aufbauen können, die den Druck der umgebenden Dampfatmosphäre übersteigen, werden modifizierte Dampfsterilisationsverfahren angewendet. Diese Verfahren arbeiten in der Regel nicht mit gesättigtem Wasserdampf (Sattdampf), sondern mit Wasserdampf/Luft-Gemischen. Aufgrund der geringeren Energiemenge der Wasserdampf/Luft-Gemische im Vergleich zu reinem Wasserdampf werden diese Verfahren im Allgemeinen entsprechend dem Standard-Sattdampfverfahren durchgeführt, was bedeutet, dass Dampfsterilisationen mit Wasserdampf/Luft-Gemischen mit längeren Sterilisationszeiten durchgeführt werden und der Sterilisationserfolg bzw. die Sterilisationszeit zum einfacheren Vergleich auf Standardbedingungen (d. h. reiner Wasserdampf bei 121 °C und 2 bar absolut) umgerechnet wird.

So beschreibt die EP 0 703 793 B1 ein Verfahren zur Herstellung von sterilen Bereitschaftspackungen mit darin eingesiegelten, insbesondere verblisterten Behältnissen, insbesondere Spritzen, welche mit Arzneimitteln gefüllt sind. Die Sterilisation erfolgt mittels Sattdampf, wobei während des Abkühlvorgangs der Druck in der Sterilisiereinrichtung mittels Druckluft erhöht wird.

Auch die WO 2009/018948 A2 beschreibt ein Sterilisationsverfahren, wobei mit dem offenbarten Verfahren Gruppen von Gegenständen sterilisiert werden sollen und die Sterilisation mittels Wasserdampf/Luft-Gemischen erfolgt.

Die EP 1 484 069 A1 betrifft eine Sterilisationsvorrichtung, welche eine Kammer zur Lagerung eines zu sterilisierenden Materials aufweist, wobei das Material mittels Dampf unter hohen Temperaturen und Druck sterilisiert werden soll. Die Sterilisationsvorrichtung weist weiterhin einen Kühlmechanismus zur Kühlung des Materials auf.

Der vorliegenden Erfindung liegt somit die Aufgabe zugrunde, ein Sterilisationsverfahren bereitzustellen, welches die zuvor geschilderten Probleme und Nachteile des Standes der Technik wenigstens teilweise vermeidet oder aber zumindest abschwächt.

Insbesondere liegt der vorliegenden Erfindung die Aufgabe zugrunde, ein Sterilisationsverfahren bereitzustellen, welches eine erhöhte Effektivität bzw. eine verkürzte Sterilisationszeit gegenüber den Verfahren des Standes der Technik aufweist.

Insbesondere soll ein Dampfsterilisationsverfahren zur Verfügung gestellt werden, welches gegenüber den bislang bekannten Verfahren eine höhere Effektivität besitzt, insbesondere mit einer verkürzten Sterilisationszeit durchgeführt werden kann, ansonsten jedoch unter "schonenden" bzw. mit dem üblichen Verfahrensverlauf vergleichbaren Bedingungen arbeitet.

Insbesondere besteht eine weitere Aufgabe der vorliegenden Erfindung darin, ein mit einem medizinischen Gut befülltes Behältnis bereitzustellen, welches gegenüber mit medizinischen Gütern befüllten Behältnissen des Standes der Technik eine reduzierte Keimzahl aufweist.

Zur Lösung der zuvor geschilderten Aufgabe schlägt die vorliegende Erfindung ein Verfahren nach Anspruch 1 vor; weitere vorteilhafte Ausgestaltungen sind Gegenstand der diesbezüglichen abhängigen Ansprüche.

Weiterhin ist bei allen nachstehend genannten relativen bzw. prozentualen Angaben zu beachten, dass diese im Rahmen der vorliegenden Erfindung vom Fachmann derart auszuwählen sind, dass in der Summe stets 100 % resultieren. Dies versteht sich für den Fachmann aber von selbst.

Denn, wie nunmehr überraschend gefunden wurde, lassen sich durch eine Druckerhöhung in einer nachgeschalteten zweiten Verfahrensstufe des Sterilisationsverfahrensschritts die Effektivität der Sterilisation und die Rate der Abtötung bzw. irreversiblen Inaktivierung von Mikroorganismen signifikant steigern, wobei die Sterilisation bei vergleichsweise milden Verfahrensbedingungen durchgeführt werden kann.

Durch die Druckerhöhung während des Sterilisationsverfahrensschrittes, insbesondere in der nachgeschalteten zweiten Verfahrensstufe, wird die Kondensation des Wasserdampfes an dem Sterilisiergut verbessert bzw. optimiert, was zu einer besseren Ausnutzung der im System befindlichen Energie durch zusätzliche Wärmeübertragung führt. Mit dem erfindungsgemäßen Verfahren kann somit bei gleicher Sterilisationstemperatur eine verbesserte Abtötung bzw. irreversible Inaktivierung der Mikroorganismen oder eine verkürzte Sterilisationszeit im Vergleich zu Dampfsterilisationsverfahren des Standes der Technik erreicht werden.

Im Rahmen der vorliegenden Erfindung ist es somit möglich, die Sterilisationszeit unter schonenden Bedingungen zu verkürzen, da mit dem erfindungsgemäßen Verfahren dieselben Sterilisationsgüter und Materialien sterilisiert werden können, wie bei Standard-Sattdampfverfahren oder Verfahren mit Wasserdampf/LuftGemischen, d. h. das erfindungsgemäße Verfahren stellt keine erhöhten Anforderungen an die Hitzebeständigkeit der zu sterilisierenden Güter bzw. Materialien.

Darüber hinaus sind dem erfindungsgemäßen Verfahren auch sämtliche Vorteile der üblichen Dampfsterilisationsverfahren innewohnend. So können mit dem erfindungsgemäßen Verfahren beispielsweise auch Güter in verschlossenen Behältnissen oder in entsprechend ausgestalteten Bereitschaftsverpackungen sterilisiert werden. Mit dem erfindungsgemäßen Verfahren entfällt somit eine aufwendige sowie zeit- und kostenintensive Befüllung der Behältnisse mit Gütern für die medizinische Anwendung unter sterilen Bedingungen.

Das erfindungsgemäße Verfahren ist darüber hinaus in hervorragender Weise reproduzierbar und kann problemlos automatisiert werden. Insbesondere ist mit dem erfindungsgemäßen Verfahren eine ständige simultane Überwachung der Sterilisation sowie eine schnelle und zielgerichtete Anpassung der Verfahrensführung an den jeweiligen Verlauf der Sterilisation möglich.

Bei der im Rahmen des erfindungsgemäßen Verfahrens eingesetzten Wasserdampf enthaltenden Sterilisationsatmosphäre kann es sich um Sattdampfatmosphären, um Atmosphären aus überhitztem Dampf oder aber um Gemische aus Gasen, insbesondere Inertgasen und/oder Luft, und Wasserdampf handeln.

Unter dem Begriff "erhöhter Druck" ist im Rahmen der vorliegenden Erfindung der Druck einer Gasphase zu verstehen, welcher höher ist als der Druck unter Standardbedingungen (1,013 bar bei 25 °C) oder als der Druck der umgebenden Atmosphäre (im Allgemeinen ungefähr 1 bar). Ein erhöhter Druck kann folglich durch einen positiven Betrag des Relativdrucks charakterisiert bzw. erfasst werden.

Unter dem "Relativdruck" eines Systems wird im Rahmen der vorliegenden Erfindung die Druckdifferenz zwischen dem Absolutdruck der Gasphase des betreffenden Systems (beispielsweise dem Druck innerhalb eines Autoklaven) und dem Absolutdruck der umgebenden Atmosphäre verstanden.

Bei einem "medizinischen Befüllungsgut" handelt es sich im Rahmen der vorliegenden Erfindung insbesondere um Arzneimittel, Pharmazeutika und Medizinprodukte. Beispielsweise sind die erfindungsgemäß eingesetzten und mit einem medizinischen Befüllungsgut befüllten Behältnisse mit Kathetergel befüllte Spritzen oder dergleichen.

Was die erste und die zweite Verfahrensstufe des Sterilisationsverfahrensschritts des erfindungsgemäßen Verfahrens anbelangt, so kann der zweite Verfahrensschritt unmittelbar auf den ersten Verfahrensschritt folgen. Es ist jedoch auch möglich, dass zwischen der ersten und der zweiten Verfahrensstufe weitere Verfahrensstufen liegen, deren jeweiliger Druck von der ersten und/oder der zweiten Verfahrensstufe verschieden sein kann.

Weiterhin ist von Bedeutung, dass im Rahmen der vorliegenden Erfindung das zu sterilisierende Behältnis vor Beginn des erfindungsgemäßen Verfahrens mit dem medizinischen Befüllungsgut befüllt wird, und zwar insbesondere unter nichtsterilen Bedingungen. Im Rahmen der vorliegenden Erfindung soll somit nicht nur die äußere Oberfläche des Behältnisses sterilisiert werden, sondern vielmehr auch das sich in dem Behältnis befindende medizinische Befüllungsgut.

Der Sterilisationsverfahrensschritt des erfindungsgemäßen Verfahrens umfasst insbesondere lediglich den Zeitraum, in welchem das Sterilisiergut die gewünschte Sterilisationstemperatur aufweist bzw. in welchem sich die Temperatur des Sterilisiergutes in dem für die Sterilisation vorbestimmten Temperaturbereich befindet.

Im Rahmen der vorliegenden Erfindung ist es vorgesehen, dass dem Sterilisationsverfahrensschritt ein Aufheizverfahrensschritt vorgeschaltet ist und dass dem Sterilisationsverfahrensschritt ein Abkühlverfahrensschritt nachgeschaltet ist. Der Aufheizverfahrensschritt umfasst dabei sowohl die eigentliche Aufheizphase (auch Steigzeit genannt), in welcher die Temperatur der Sterilisationsatmosphäre erreicht wird, als auch die so genannte Ausgleichszeit, die zusätzlich zu der Aufheizphase benötigt wird. Die Ausgleichszeit (synonym auch als "Plateauzeit" bezeichnet) beschreibt bzw. definiert den Zeitraum, welcher bis zur vollständigen Durchwärmung des Sterilisationsgutes auf die gewünschte Sterilisationstemperatur benötigt wird.

Im Rahmen der vorliegenden Erfindung kann es vorgesehen sein, dass der Sterilisationsverfahrensschritt in Gegenwart einer reinen Wasserdampf enthaltenden Atmosphäre und/oder als Sattdampfverfahren durchgeführt wird. Unter Sattdampf ist dabei eine gesättigte Atmosphäre aus reinem Wasserdampf zu verstehen (wobei reiner Wasserdampf, insbesondere nach der europäischen Norm EN 285, bis zu 3,5 Vol.-% nicht kondensierbare Gase enthalten kann, wie Sauerstoff, Stickstoff etc.), während eine reinen Wasserdampf enthaltenden Atmosphäre beispielsweise auch eine Atmosphäre aus überhitztem Dampf sein kann.

Besonders gute Ergebnisse werden im Rahmen der vorliegenden Erfindung insbesondere dann erzielt, wenn der Sterilisationsverfahrensschritt in Gegenwart eines Wasserdampf/Gas-Gemisches, insbesondere eines Gemisches von Wasserdampf mit Stickstoff und/oder Sauerstoff und/oder Inertgas, vorzugsweise eines Wasserdampf/Luft-Gemisches, durchgeführt wird. Die Erzeugung von Wasserdampf/GasGemischen kann dabei durch getrennte oder gemeinsame Zugabe von Gas und Wasserdampf vorgenommen werden, wobei die Zusammensetzung der Wasserdampf/Gas-Gemische, d. h. die jeweiligen Anteile an Wasserdampf und Gas bzw. Luft, unkritisch ist und nahezu beliebig variiert werden kann. Bei Wasserdampf/LuftGemischen ist auf eine innige, vorzugsweise ständige Umwälzung bzw. Verwirbelung zu achten, da sich diese andernfalls entmischen, wodurch der Erfolg der Sterilisation gefährdet würde.

Im Allgemeinen wird das Verfahren in einer geschlossenen Sterilisationseinrichtung, insbesondere in einem gasdicht verschlossenen Druckbehälter, vorzugsweise in einer Autoklaviereinrichtung (Autoklav), durchgeführt.

In diesem Zusammenhang hat es sich als vorteilhaft erwiesen, wenn die Sterilisationsatmosphäre durch Injektion von Wasserdampf bzw. Wasserdampf/Druckluft-Gemischen, vorzugsweise Wasserdampf/Druckluft-Gemischen, in die Sterilisationseinrichtung erzeugt wird.

Hierbei hat es sich als vorteilhaft erwiesen, wenn die Sterilisationsatmosphäre durch Injektion von bereits vorgemischten Wasserdampf/Druckluft-Gemischen oder durch jeweils separate Injektionen von Wasserdampf und Druckluft in die Sterilisationseinrichtung erzeugt wird. Die Injektion von bereits vorgemischten Wasserdampf/Druckluft-Gemischen ist dabei technisch einfacher durchzuführen, erlaubt jedoch eine nur eingeschränkte Anpassung an den jeweiligen Verfahrensverlauf. Die separate Injektion von Wasserdampf und Gas bzw. Druckluft hat den Vorteil, dass die einzelnen Parameter der Sterilisationsatmosphäre, wie Druck, Temperatur oder Zusammensetzung, besser und einfacher einstellbar sowie einzeln regelbar sind, so dass auf den jeweiligen Verfahrensverlauf individuell reagiert werden kann und das Verfahren insgesamt an verschiedene Anforderungsprofile problemlos angepasst werden kann. Erfindungsgemäß wird daher die separate Injektion von Wasserdampf und Druckluft bzw. Gas in die Sterilisationseinrichtung bevorzugt.

Gleichermaßen hat es sich im Rahmen der vorliegenden Erfindung als vorteilhaft erwiesen, wenn der Wasserdampf aus destilliertem und/oder vollentsalztem Wasser erzeugt wird bzw. wenn die Druckluft aus steriler Luft erzeugt wird. Dabei ist es Vorteilhafterweise insbesondere vorgesehen, dass die Luft mittels Durchströmen von Filtern gereinigt bzw. sterilisiert wird. Als Filter kommen dabei insbesondere Partikelfilter zum Einsatz, welche Objekte in der Größenordnung von im Allgemeinen kleiner 1 µm, insbesondere aber größer als 0,2 µm, abscheiden können, wie beispielsweise HEPA-Filter (High Efficiency Particulate Air Filter), ULPA-Filter (UItra Low Penetration Air Filter) und SULPA-Filter (Super Ultra Low Penetration Air Filter). Die Verwendung sterilisierter Luft hat den Vorteil, dass keine zusätzlichen Mikroorganismen in die Sterilisiereinrichtung eingetragen werden und eine Kontamination des Sterilisierguts unter Erhöhung der Ausgangskeimzahl ausgeschlossen wird. Die Verwendung von vollentsalztem bzw. destilliertem Wasser zur Erzeugung des Wasserdampfes schont einerseits die Sterilisationseinrichtung sowie deren Zuund Ableitungen und verhindert andererseits die Ablagerung von Rückständen bzw. Salzen auf dem Sterilisiergut.

Im Allgemeinen wird der Sterilisationsverfahrensschritt in einem Temperaturbereich zwischen 100 und 180 °C, insbesondere 100 bis 150 °C, vorzugsweise 105 bis 145 °C, besonders bevorzugt 105 bis 140 °C, ganz besonders bevorzugt 110 bis 135 °C, noch mehr bevorzugt 110 bis 130 °C, durchgeführt. In diesen Temperaturbereichen kann das erfindungsgemäße Sterilisationsverfahren wirkungsvoll in annehmbaren Zeiten durchgeführt werden und ist für eine Vielzahl von Materialien geeignet.

Im Allgemeinen wird der Sterilisationsverfahrensschritt über eine Zeitdauer von 0,1 bis 600 Minuten, insbesondere 0,5 bis 300 Minuten, vorzugsweise 1 bis 100 Minuten, bevorzugt 2 bis 60 Minuten, besonders bevorzugt 3 bis 45 Minuten, noch mehr bevorzugt 4 bis 30 Minuten, ganz besonders bevorzugt 5 bis 20 Minuten, am meisten bevorzugt 5 bis 15 Minuten, durchgeführt. Mit dem erfindungsgemäßen Verfahren ist es somit möglich, die Sterilisation mit im Vergleich zum Standard-Sattdampfverfahren verkürzten Sterilisationszeiten durchzuführen - und dies bei vergleichbaren Temperaturen.

Was die Zeitdauer der einzelnen Verfahrensstufen des Sterilisationsverfahrensschrittes anbelangt, so hat es sich im Rahmen der vorliegenden Erfindung als vorteilhaft erwiesen, wenn 20 bis 95 %, insbesondere 40 bis 90 %, vorzugsweise 50 bis 85 %, besonders bevorzugt 60 bis 80 %, der Zeitdauer des Sterilisationsverfahrensschrittes auf die erste Verfahrensstufe des Sterilisationsverfahrensschrittes entfallen bzw. wenn 5 bis 80 %, insbesondere 10 bis 60 %, vorzugsweise 5 bis 50 %, besonders bevorzugt 20 bis 40 %, der Zeitdauer des Sterilisationsverfahrensschritts auf die zweite Verfahrensstufe des Sterilisationsverfahrensschritts entfallen.

Erfindungsgemäß ist es vorgesehen, dass der Sterilisationsverfahrensschritt in einem Relativdruckbereich von 0,05 bis 10 bar, insbesondere 0,1 bis 4 bar, vorzugsweise 0,5 bis 3,5 bar, besonders bevorzugt 1 bis 3,25 bar, noch mehr bevorzugt 1,5 bar bis 3 bar, ganz besonders bevorzugt 2 bar bis 2,8 bar, durchgeführt wird. Anders ausgedrückt wird der Sterilisationsverfahrensschritt in einem Absolutdruckbereich von 1,05 bis 11 bar, insbesondere 1,1 bis 5 bar, vorzugsweise 1,5 bis 4,5 bar, besonders bevorzugt 2 bis 4,25 bar, noch mehr bevorzugt 2,5 bar bis 4 bar, ganz besonders bevorzugt 3 bar bis 3,8 bar, durchgeführt werden. In diesen Druckbereichen wird eine ausreichende Kondensation des Wasserdampfes auf dem Sterilisiergut gewährleistet und können beispielsweise Wasserdampf/LuftGemische eingesetzt werden, die sich in besonderer Weise zur effektiven Sterilisation eignen.

Bei der Durchführung des erfindungsgemäßen Verfahrens hat es sich als vorteilhaft erwiesen, wenn der Relativdruck zu Beginn des Sterilisationsverfahrensschritts auf einen Wert von mindestens 1 bar, insbesondere mindestens 1,2 bar, vorzugsweise mindestens 1,5 bar, bevorzugt mindestens 2 bar, eingestellt wird. Insbesondere kann der Absolutdruck zu Beginn des Sterilisationsverfahrensschritts auf einen Wert von mindestens 2 bar, insbesondere mindestens 2,2 bar, vorzugsweise mindestens 2,5 bar, bevorzugt mindestens 3 bar, eingestellt werden. Bei diesen Drücken ist eine sehr gute Wärmeübertragung auf das Sterilisiergut durch den Wasserdampf gegeben, so dass hervorragende Sterilisationsergebnisse bei insgesamt kurzer Sterilisationszeit erreicht werden können.

Die genaue Einstellung des Drucks während des Sterilisationsverfahrensschritts, insbesondere zu Beginn des Sterilisationsverfahrensschritts, ist dabei von den jeweiligen Verfahrensbedingungen, wie der Zusammensetzung der Sterilisationsatmosphäre oder den zu sterilisierenden Materialien, abhängig: Wird in der ersten Stufe des Sterilisationsverfahrensschritts eine Sterilisationsatmosphäre aus gesättigtem Wasserdampf eingesetzt, so hat es sich als vorteilhaft erwiesen, wenn der Relativdruck zu Beginn des Sterilisationsverfahrensschritts im Bereich von 1 bis 1,5 bar eingestellt wird bzw. wenn der Absolutdruck zu Beginn des Sterilisationsverfahrensschritts im Bereich von 2 bis 2,5 bar eingestellt wird.

Wird hingegen in der ersten Verfahrensstufe des Sterilisationsverfahrensschritts ein Wasserdampf/Gas-Gemisch, insbesondere Wasserdampf/Luft-Gemisch, eingesetzt, so kann der Druck in weiten Bereichen variieren, insbesondere in den zuvor genannten allgemeinen Bereichen.

Neben dem Betrag des Drucks beeinflusst jedoch auch die Dauer der jeweiligen Druckeinwirkung den Verfahrensablauf.

So kann es im Rahmen der vorliegenden Erfindung vorgesehen sein, dass der Relativdruck während des Sterilisationsverfahrensschritts einen Wert von 1 bar, insbesondere 1,2 bar, vorzugsweise 1,5 bar, bevorzugt 2 bar, zumindest zeitweise überschreitet, insbesondere für eine Zeitdauer von mindestens 10 %, insbesondere mindestens 20 %, vorzugsweise mindestens 30 %, besonders bevorzugt mindestens 40 %, ganz besonders bevorzugt mindestens 50 %, der Gesamtdauer des Sterilisationsverfahrensschritts und am meisten bevorzugt für die Gesamtdauer des Sterilisationsverfahrensschritts.

Somit kann es vorgesehen sein, dass der Absolutdruck während des Sterilisationsverfahrensschritts einen Wert von 2 bar, insbesondere 2,2 bar, vorzugsweise 2,5 bar, bevorzugt 3 bar, zumindest zeitweise überschreitet, insbesondere für eine Zeitdauer von mindestens 10 %, insbesondere mindestens 20 %, vorzugsweise mindestens 30 %, besonders bevorzugte mindestens 40 %, ganz besonders bevorzugt mindestens 50 %, der Gesamtdauer des Sterilisationsverfahrensschritts und am meisten bevorzugt für die Gesamtdauer des Sterilisationsverfahrensschritts.

Im Rahmen der vorliegenden Erfindung wird es bevorzugt, wenn die vorgenannten Drücke und Zeitintervalle eingehalten werden, da dies eine verlässliche Abtötung der Keime - bedingt durch die hohe Temperatur und die gute Wärmeübertragung - bei praktikablen bzw. annehmbaren Verfahrensdauern ermöglicht. Dabei ist es besonders bevorzugt, wenn die genannten Drücke über die gesamte Dauer des Sterilisationsverfahrensschritts aufrechterhalten werden.

Besonders gute Ergebnisse werden im Rahmen der vorliegenden Erfindung erhalten, wenn der Relativdruck bei Abschluss des Sterilisationsverfahrensschritts auf Werte von weniger als 4 bar, insbesondere weniger als 3,5 bar, vorzugsweise weniger als 3,25 bar, bevorzugt weniger als 3 bar, ganz besonders bevorzugt weniger als 2,8 bar, eingestellt wird bzw. wenn der Absolutdruck bei Abschluss des Sterilisationsverfahrensschritts auf Werte von weniger als 5 bar, insbesondere weniger als 4,5 bar, vorzugsweise weniger als 4,25 bar, bevorzugt weniger als 4 bar, ganz besonders bevorzugt weniger als 3,8 bar, eingestellt wird.

Im Allgemeinen beträgt die relative Druckerhöhung von der ersten zu der zweiten Verfahrensstufe des Sterilisationsverfahrensschritts mindestens 0,01 bar, insbesondere mindestens 0,05 bar, vorzugsweise mindestens 0,1 bar, noch mehr bevorzugt mindestens 0,15 bar, besonders bevorzugt mindestens 0,2 bar, ganz besonders bevorzugt mindestens 0,25 bar.

In diesem Zusammenhang hat es sich als besonders vorteilhaft erwiesen, wenn der relative Druck von der ersten zu der zweiten Verfahrensstufe des Sterilisationsverfahrensschritts im Bereich von 0,01 bis 2 bar, insbesondere im Bereich von 0,05 bis 1 bar, vorzugsweise im Bereich von 0,1 bis 0,75 bar, bevorzugt im Bereich von 0,15 bis 0,5 bar, noch mehr bevorzugt im Bereich von 0,2 bis 0,4 bar, erhöht wird. In diesem Druckbereich schlägt sich die Druckerhöhung maßgeblich in einer gesteigerten Effektivität und verkürzten Dauer der Sterilisation nieder, erlaubt jedoch gleichermaßen noch eine wirtschaftlich sinnvolle Durchführung des Verfahrens und belastet darüber hinaus das Sterilisiergut bzw. die eingesetzten Materialien nicht übermäßig.

Erfindungsgemäß erfolgt die Druckerhöhung von der ersten zu der zweiten Verfahrensstufe des Sterilisationsverfahrensschritts mittels Eintrag von Luft, vorzugsweise Druckluft. Hierbei ist es wiederum von Vorteil, wenn die Luft, vorzugsweise die Druckluft, steril ist.

Eine Druckerhöhung durch Luft, vorzugsweise Druckluft in der zweiten Verfahrensstufe des Sterilisationsverfahrensschritts bzw. beim Übergang von der ersten zu der zweiten Verfahrensstufe ermöglicht in besonderer Weise die optimale Ausnutzung der im Wasserdampf enthaltenen Energie durch eine verbesserte bzw. optimierte Kondensation des Wasserdampfes, da gleichzeitig der zusätzliche Energieeintrag in die Sterilisiereinrichtung durch das Gas bzw. die Druckluft im Vergleich zu Wasserdampf nur äußerst gering ist. Eine optimale Ausnutzung der durch die Druckerhöhung gewonnenen Vorteile wird somit durch die Druckbeaufschlagung mittels Gas, insbesondere Luft, vorzugsweise Druckluft verwirklicht.

Im Rahmen des erfindungsgemäßen Verfahrens kann es vorgesehen sein, dass der Sterilisationsverfahrensschritt 2 bis 10 Verfahrensstufen umfasst, wobei mindestens 2 Verfahrensstufen bei unterschiedlichen Drücken durchgeführt werden. Dabei kann es insbesondere vorgesehen sein, dass der Druck in einer nachgeschalteten Verfahrensstufe gegenüber dem Druck in einer vorgeschalteten Verfahrensstufe erhöht wird.

Im Rahmen der vorliegenden Erfindung ist es besonders bevorzugt, wenn der Sterilisationsverfahrensschritt aus lediglich zwei Verfahrensstufen besteht, wobei die Druckerhöhung von der ersten zu der zweiten Verfahrensstufe durch eine Druckerhöhung mittels Druckluft, insbesondere in Form eines oder mehrerer Druckluftstöße, vorzugsweise eines Druckluftstoßes, vorgenommen wird. Der Einsatz von Druckluftstößen, insbesondere eines einzigen Druckluftstoßes, ermöglicht eine schnelle Erhöhung des Druckes innerhalb der Sterilisationseinrichtung und führt zu einer sofort einsetzenden verbesserten Kondensation des Dampfes. Vorzugsweise werden der Druckluftstoß bzw. die Druckluftstöße mit Druckluft durchgeführt, wobei die Temperatur der Sterilisationsatmosphäre nach dem Einleiten der Druckluft durchaus fallen darf; es jedoch unerlässlich ist, dass die Temperatur des Sterilisationsgutes für die beabsichtigte Sterilisationsdauer auf der eingestellten Sterilisationstemperatur bleibt bzw. sich in dem für die Sterilisation gewünschten und erforderlichen Temperaturintervall (d. h. dem vorgegebenen Temperaturband oder Temperaturregime) befindet.

Im Rahmen der vorliegenden Erfindung können sehr gute Ergebnisse erhalten werden, wenn die erste Verfahrensstufe des Sterilisationsverfahrensschritts in Form eines SattdampfVerfahrens durchgeführt wird und die Druckbeaufschlagung bzw. Druckerhöhung von der ersten zu der zweiten Verfahrensstufe mittels steriler Druckluft erfolgt. Diese spezielle Verfahrensführung eignet sich beispielsweise für die Sterilisation von mit einem medizinischen Befüllungsgut befüllten Behältnissen, in welchen sich während der Sterilisation kein hoher Druck aufbaut oder welche einem hohen Innendruck unbeschadet widerstehen können.

Darüber hinaus werden im Rahmen der vorliegenden Erfindung sehr gute Ergebnisse erhalten, wenn die erste Verfahrensstufe des Sterilisationsverfahrensschritts in einer Sterilisationsatmosphäre aus einem Wasserdampf/Gas-Gemisch, insbesondere einem Wasserdampf/Luft-Gemisch, durchgeführt wird und die Druckbeaufschlagung bzw. Druckerhöhung von der ersten zu der zweiten Verfahrensstufe mittels steriler Druckluft erfolgt. Dies Art der Verfahrensführung ermöglicht eine sehr viel breitere und umfangreichere Anwendung des erfindungsgemäßen Verfahrens als der Einsatz einer Sterilisationsatmosphäre aus gesättigtem Wasserdampf in der ersten Verfahrensstufe des Sterilisationsverfahrensschritts, da einem Druckaufbau im Inneren der zu sterilisierenden Behältnisse entgegengewirkt werden kann. Beispielsweise könne bei Verwendung einer Sterilisationsatmosphäre aus einem Wasserdampf/Luft-Gemisch mit Flüssigkeiten befüllte Spritzen sterilisiert werden.

Im Allgemeinen wird bei der Durchführung des erfindungsgemäßen Verfahrens derart vorgegangen, dass das Verfahren zumindest während des Sterilisationsverfahrensschritts, vorzugsweise während der gesamten Verfahrensdauer, über die Messung und Regelung von Druck und/oder Temperatur (insbesondere Messung und Regelung ausschließlich von Druck und/oder Temperatur), vorzugsweise Druck und Temperatur, insbesondere Druck und Temperatur der Sterilisationsatmosphäre und gegebenenfalls der Temperatur mindestens einer in der Sterilisationseinrichtung befindlichen Referenzprobe, insbesondere mehrerer in der Sterilisationseinrichtung befindlicher Referenzproben, gesteuert bzw. kontrolliert wird. Grundsätzlich kann das erfindungsgemäße Verfahren auch ohne derartige Referenzproben durchgeführt werden. Erfindungsgemäß bevorzugt verläuft die Steuerung und/oder Kontrolle des Verfahrens ausschließlich über die Messung und Regelung mindestens einer der beiden vorgenannten Parameter (d. h. Druck und/oder Temperatur).

Bislang war es im Stand der Technik - wenn überhaupt - üblich, Temperatur und Feuchtigkeit der Sterilisationsatmosphäre zu bestimmen, wobei gegebenenfalls noch die Temperatur von Referenzproben bestimmt wurde. Gerade die Feuchtigkeitsmessung stellt sich jedoch als besonders unpräzise und messtechnisch schwierig zu kontrollieren heraus und führt oftmals zu falschen Werten.

Unter einer Referenzprobe ist im Rahmen der vorliegenden Erfindung insbesondere eine Kontrollprobe zu verstehen, bei der es sich um ein vorzugsweise baugleiches oder zumindest ähnliches bzw. vergleichbares (d. h. korrelierbares) Behältnis zu dem Sterilisiergut mit dem gleichen Befüllungsgut handelt, welches jedoch mit einem Sensor bzw. einer Einrichtung zur Messung bzw. Bestimmung der Temperatur des Befüllungsguts versehen ist.

Darüber hinaus hat es sich im Rahmen der vorliegenden Erfindung als vorteilhaft erwiesen, wenn zumindest während des Sterilisationsverfahrensschritts, vorzugsweise während des gesamten Verfahrens, Druck und/oder Temperatur, vorzugsweise Druck und Temperatur, insbesondere Druck und Temperatur der Sterilisationsatmosphäre und gegebenenfalls die Temperatur mindestens einer in der Sterilisationseinrichtung befindlichen Referenzprobe, insbesondere mehrerer in der Sterilisationseinrichtung befindlicher Referenzproben, bestimmt und gegebenenfalls eingestellt werden, insbesondere auf vorgegebene Werte.

Besonders gute Ergebnisse werden erhalten, wenn die Druck- und Temperaturdaten zur Kontrolle und/oder Steuerung zumindest des Sterilisationsverfahrensschritts, vorzugsweise des gesamten Verfahrens, verwendet werden.

Die ermittelten Daten erlauben zum einen eine nachträgliche Überprüfung des Sterilisationsvorgangs und eine adäquate Archivierung der Ergebnisse; es ist jedoch auch eine Simultanüberwachung des Sterilisationsvorgangs möglich, so dass eine schnelle Anpassung der Verfahrensführung aufgrund der ermittelten Messwerte erfolgen kann. So kann das Verfahren individuell und flexibel einerseits an spezifische Gegebenheiten der Produkte angepasst werden; andererseits ist auch eine schnelle Reaktion auf während des Sterilisationsverfahrens auftretende Probleme möglich, so dass diese bereits während der Sterilisation behoben werden können. Auf diese Weise wird die Gefahr von unzureichenden Sterilisationen mit dem einhergehenden hohen wirtschaftlichen Verlust vermieden bzw. minimiert.

Im Allgemeinen wird das erfindungsgemäße Verfahren entsprechend dem Standard-Sattdampfverfahren, insbesondere als so genanntes F₀-Wert-Verfahren, durchgeführt bzw. wird das Verfahren in Form eines Overkill-Verfahrens durchgeführt. Alternativ kann das erfindungsgemäße Verfahren jedoch auch produktspezifisch unter Berücksichtigung einer zu erwartenden und/oder einer experimentell bestimmten Keimanzahl und/oder Keimart durchgeführt werden.

Beim Standard-Sattdampfverfahren wird das Sterilisiergut 15 Minuten auf 121 °C erhitzt, wodurch eine Reduktion der Ausgangskeimzahl von 10⁴ auf einen Wert von 10⁻⁶ erfolgt, was dem Sterility Assurance Level (SAL) entspricht. In der Sterilisationszeit von 15 Minuten ist auch bereits ein Sicherheitszuschlag enthalten, welcher gewährleisten soll, dass der Sterilisationserfolg tatsächlich erreicht wird.

Bei den Äquivalenz-Verfahren bzw. den entsprechend dem Standard-Sattdampfverfahren durchgeführten Sterilisationsverfahren wird der so genannte F₀-Wert berechnet, welcher angibt, wie viele Minuten ein Objekt äquivalent zu 121 °C sterilisiert wird. Auch bei den Äquivalenz-Verfahren bzw. den entsprechend dem Standard-Sattdampfverfahren durchgeführten Sterilisationsverfahren soll eine Keimreduktion von 10⁴ auf 10⁻⁶ pro Einheit des Sterilisierguts erreicht werden, wobei jedoch abweichend vom Standardverfahren beispielsweise eine andere Temperatur gewählt wird oder durch die Verwendung von Wasserdampf/Luft-Gemischen ein anderer Betrag bei der Energieübertragung erzielt wird. Die Umrechnung auf Standardbedingungen und die Angabe des F₀-Wertes ermöglichen einen sofortigen und einfachen Vergleich bzw. eine Beurteilung des Sterilisationserfolges, wobei die zur Umrechnung benötigten Daten bzw. Umrechnungsfaktoren in Abhängigkeit von der genauen Verfahrensdurchführung entweder entsprechenden Nachschlagewerken entnommen werden können oder aber experimentell bestimmt werden müssen. Sowohl beim Standard-Sattdampfverfahren als auch bei entsprechenden F₀-Wert-Verfahren, wie dem Äquivalenz-Verfahren, muss der Sterilisationserfolg durch regelmäßige Kontrollen nachgewiesen bzw. dokumentiert werden.

Das Overkill-Verfahren unterscheidet sich vom Standard- bzw. Äquivalenz-Verfahren dadurch, dass eine Ausgangskeimzahl von 10⁶ je Einheit des Sterilisierguts angenommen wird, also eine 100-mal höhere Keimbelastung als beim Standard- bzw. Äquivalenz-Verfahren, welche auf einen Wert von 10⁻⁶ reduziert werden soll. Dieser Sicherheitszuschlag durch Annahme einer höheren Keimbelastung ermöglicht größere Intervalle bei der Bestimmung der tatsächlichen Keimbelastung bzw. der tatsächlich auf dem Sterilisiergut vorkommenden Keime sowie des Sterilisationserfolges.

Bei der produktspezifischen Durchführung des erfindungsgemäßen Verfahrens werden experimentell die auf dem nicht sterilisierten Sterilisiergut anfangs zu erwartende Keimzahl sowie die zu erwartenden Keimarten bestimmt und anhand dieser Daten das Verfahren derart ausgeführt, dass eine Reduktion auf eine Keimbelastung von 10⁻⁶ erreicht wird. Auf diese Weise können deutlich kürzere Sterilisationszeiten erreicht werden; es werden jedoch hohe Anforderungen an die ständige Kontrolle der Keimarten bzw. Keimzahlen auf dem Sterilisiergut, das so genannte Bio-Burden-Monitoring, gestellt.

Wie zuvor ausgeführt, umfasst das erfindungsgemäße Verfahren einen Aufheizverfahrensschritt.

In diesem Zusammenhang hat es sich im Rahmen der vorliegenden Erfindung bewährt, wenn während des Aufheizverfahrensschritts Druck und/oder Temperatur, insbesondere Druck und Temperatur, der Atmosphäre innerhalb der Sterilisationseinrichtung geändert, insbesondere erhöht, werden, insbesondere kontinuierlich und/oder diskontinuierlich geändert werden.

In diesem Zusammenhang werden besonders gute Ergebnisse erhalten, wenn der Aufheizverfahrensschritt in mindestens 2 Verfahrensstufen, vorzugweise in 2 bis 10 Verfahrensstufen, unterteilt ist und der Druck in der Sterilisationseinrichtung einer nachgeschalteten Verfahrensstufe gegenüber dem Druck in einer unmittelbar vorgeschalteten Verfahrensstufe insbesondere durch Evakuierung gesenkt oder insbesondere durch Eintrag von Wasserdampf und Gas, insbesondere Druckluft, erhöht wird.

Während des Aufheizverfahrensschritts können Druck und Temperatur in beliebiger Reihenfolge bzw. Abfolge erhöht oder erniedrigt werden, insbesondere mit der Maßgabe, dass bei Ende des Aufheizverfahrensschritts Druck und Temperatur den Bedingungen für den eigentlichen Sterilisationsverfahrensschritt entsprechen.

Im Allgemeinen wird während des Aufheizverfahrensschritts die in der ersten Verfahrensstufe des Sterilisationsverfahrensschritts eingesetzte Sterilisationsatmosphäre erzeugt. Auf diese Weise kann sich der Sterilisationsverfahrensschritt unmittelbar, d. h. ohne Zwischenstufen, an den Aufheizverfahrensschritt anschließen.

Im Rahmen der vorliegenden Erfindung umfasst der Aufheizverfahrensschritt insbesondere auch die so genannte Ausgleichszeit, d. h. die Zeit, welche zur vollständigen Durchwärmung des Sterilisierguts benötigt wird, nachdem die Temperatur in der Sterilisationseinrichtung bereits den vorgegebenen Wert für den Sterilisationsverfahrensschritt erreicht hat. Im Rahmen der vorliegenden Erfindung umfasst der Aufheizverfahrensschritt somit die Steigzeit und die Ausgleichszeit.

Zum Aufheizen der Sterilisationseinrichtung sowie zur Erzeugung der Sterilisationsatmosphäre ist dem Fachmann eine Vielzahl von Verfahren bekannt: So kann beispielsweise mittels des Gravitationsverfahrens die schwerere Luft gegen leichteren heißen Wasserdampf ausgetauscht werden. Eine höhere Reinheit der Sterilisationsatmosphäre wird jedoch durch Vakuumverfahren erreicht. Im einfachsten Fall besteht ein solches Vakuumverfahren aus einem einfachen Vorvakuum und einer anschließenden Druckerhöhung mittels Wasserdampf oder eines Wasserdampf/Luft-Gemisches. Noch bessere Ergebnisse werden jedoch durch ein fraktioniertes Vakuum erreicht, wobei die fraktionierten Vakuumverfahren in subatmosphärische, superatmosphärische und transatmosphärische Vakuumverfahren unterteilt werden. Die fraktionierten Vakuumverfahren können darüber hinaus noch mit Vakuum-Überdruckzyklen kombiniert werden, bei welchen der Druck erst erhöht wird, dann wieder abgesenkt wird, um darauf folgend auf einen höheren Wert als bei der vorangehenden Druckerhöhung erhöht zu werden, so dass insgesamt ein Druckanstieg resultiert.

Im Rahmen der vorliegenden Erfindung wird bevorzugt ein fraktioniertes Vakuum, gegebenenfalls in Kombination mit Vakuum-Überdruckzyklen, eingesetzt. Dabei ist es weiterhin bevorzugt, wenn die einzelnen Druckerhöhungen während der fraktionierten Vakuumverfahren sowie gegebenenfalls während der Vakuum-Überdruckzyklen mittels Sattdampf erfolgen und diese Verfahren derart durchgeführt werden, dass mindestens 95 % der ursprünglichen Atmosphäre ausgetauscht sind.

Im Allgemeinen ist es bei der erfindungsgemäßen Verfahrensführung üblich, dass während des Abkühlverfahrensschritts eine Abkühlung und Trocknung des mit einem medizinischen Befüllungsgut befüllten Behältnisses durchgeführt wird bzw. dass während des Abkühlverfahrensschritts Druck und/oder Temperatur, insbesondere Druck und Temperatur, der Atmosphäre innerhalb der Sterilisationseinrichtung geändert, insbesondere gesenkt, werden, insbesondere kontinuierlich und/oder diskontinuierlich geändert werden.

In diesem Zusammenhang kann es vorgesehen sein, dass der Abkühlverfahrensschritt in mindestens 2 Verfahrensstufen, vorzugsweise in 2 bis 10 Verfahrensstufen, unterteilt ist.

Wie gleichermaßen zuvor ausgeführt, umfasst das erfindungsgemäße Verfahren einen Abkühlverfahrensschritt.

Erfindungsgemäß wird während des Abkühlverfahrensschritts zumindest vorübergehend ein erhöhter Druck, insbesondere ein Stützdruck, angewendet derart, dass dem in dem mit einem medizinischen Befüllungsgut befüllten Behältnis vorhandenen Druck entgegengewirkt wird.

Unter einem Stützdruck soll dabei im Rahmen der vorliegenden Erfindung insbesondere eine zusätzliche Druckbeaufschlagung während des Abkühlverfahrensschritts, vorzugsweise durch Druckluft, verstanden werden, welche einer Expansion des noch heißen Befüllungsguts bzw. des im Behältnis vorhandenen Gases entgegenwirkt, während die Temperatur der Atmosphäre in der Sterilisationseinrichtung bereits sinkt und eine Druckerniedrigung induziert.

Vorteilhafterweise wird der Abkühlverfahrensschritt derart durchgeführt, dass Druck und Temperatur im Wechsel erhöht und gesenkt werden, so dass das kondensierte Wasser wieder in die Gasphase überführt wird und aus der Sterilisationseinrichtung entfernt werden kann. Zu diesem Zweck wird beispielsweise die Temperatur in der Sterilisationseinrichtung kurzzeitig erhöht. Der Abkühlverfahrensschritt wird somit komplementär zu den gleichfalls bevorzugten Vakuum-Überdruckzyklen während des Aufheizverfahrensschritts durchgeführt, wobei jedoch durch den zusätzlichen Stützdruck einer Expansion des Befüllungsguts entgegengewirkt wird.

Am Ende des Abkühlverfahrensschritts, insbesondere nach Erreichen einer vorgegebenen Endtemperatur, wird das sterilisierte, mit dem medizinischen Befüllungsgut befüllte Behältnis vorteilhafter Weise einer Druckbeaufschlagung unterzogen, insbesondere mit einem Relativdruck im Bereich von 0,5 bis 10 bar, insbesondere 1 bis 5 bar, vorzugsweise 1,5 bis 3 bar. Insbesondere bei Spritzen, wie Kolbenspritzen, wird hierdurch gewährleistet, dass einem reduzierten druck im Innern des Behältnisses wirksam entgegengewirkt wird.

Im speziellen Fall einer Kolbenspritze wird auf diese Weise zudem der Kolben in eine anwendungsbereite Position gebracht, so dass Anwendungsfehler vermieden werden können.

Im Rahmen der vorliegenden Erfindung ist es vorgesehen, dass das medizinische Befüllungsgut flüssig und/oder fließfähig ist. Im Rahmen der vorliegenden Erfindung kommen somit bevorzugt flüssigkeitsbasierte Befüllungsgüter, wie beispielsweise Flüssigkeiten, Gele oder Pasten, zum Einsatz. Diese Art des Befüllungsguts stellt jedoch besondere Herausforderungen an den Verfahrensablauf, da die Befüllungsgüter zu einem Druckaufbau innerhalb des Behältnisses durch teilweises Verdampfen der Flüssigkeit oder durch Gaseinschlüsse beitragen können. Aus diesem Grund wird im Rahmen der vorliegenden Erfindung eine Sterilisation mittels Wasserdampf/Luft-Gemischen bevorzugt und ist während des Abkühlverfahrensschritts die Anwendung eines Stützdrucks vorteilhaft.

Gleichfalls kann es vorgesehen sein, dass das medizinische Befüllungsgut ein Arzneimittel, Pharmazeutikum oder Medizinprodukt, insbesondere ein Gleitgel, vorzugsweise Kathetergleitgel, ist.

Im Rahmen der vorliegenden Erfindung ist das eingesetzte Behältnis ein medizinisches Behältnis, insbesondere eine Ampulle, eine Tube oder ein medizinisches Instrument, insbesondere ein Katheter oder eine Spritze, vorzugsweise eine Faltenbalg-Spritze oder eine Kolbenspritze, bevorzugt eine Kolbenspritze.

Im Rahmen der vorliegenden Erfindung werden besonders gute Sterilisationsergebnisse erhalten, wenn das Behältnis eine Kolbenspritze, insbesondere eine Einweg-Kolbenspritze, bevorzugt aus Kunststoff, vorzugsweise eine Kolbenspritze mit einem zylindrischen Hohlraum oder Grundkörper, welcher an einem Ende eine Öffnung zur Aufnahme eines Kolbens und an dem gegenüberliegenden Ende eine Düse aufweist, ist.

Gemäß einer erfindungsgemäß bevorzugten Ausführungsform ist das mit einem medizinischen Befüllungsgut befüllte Behältnis eine mit einem Kathetergleitgel befüllte Kolbenspritze, bevorzugt Einweg-Kolbenspritze, vorzugsweise aus Kunststoff.

Das Behältnis, insbesondere medizinische Behältnis, vorzugsweise die Spritze, welche(s) im Rahmen der vorliegenden Erfindung eingesetzt wird, kann aus einer Vielzahl von Materialien, insbesondere Kunststoffmaterialien, aber auch glashaltigen Materialien, bestehen, an welche lediglich die Bedingung gestellt wird, für die medizinische Anwendung geeignet und zugelassen zu sein und darüber hinaus unter Verfahrensbedingungen, insbesondere unter erhöhtem Druck und erhöhter Temperatur, stabil zu sein.

Im Rahmen der vorliegenden Erfindung hat es sich als besonders vorteilhaft erwiesen, wenn das Behältnis, insbesondere das medizinische Behältnis, vorzugsweise eine Spritze, aus hitzeresistenten Materialien besteht, insbesondere aus Materialien, welche Temperaturen im Bereich von 100 bis 150 °C, insbesondere Temperaturen bis 140 °C, vorzugsweise bis 135 °C, bevorzugt bis 130 °C, widerstehen. Dabei hat es sich als besonders vorteilhaft erwiesen, wenn das Behältnis, insbesondere das medizinische Behältnis, vorzugsweise eine Spritze, teilweise oder vollständig, bevorzugt vollständig, aus hitzeresistenten Kunststoffmaterialien oder Glasmaterialien besteht.

Gemäß einer besonderen Ausführungsform der vorliegenden Erfindung kann es vorgesehen sein, dass eine Vielzahl von mit einem medizinischen Befüllungsgut befüllten Behältnissen gleichzeitig sterilisiert werden; dabei können während des Verfahrens mehrere Behältnisse auf einem Träger zusammengeführt oder zu einer Einheit zusammengefasst sein. Diese Art der Durchführung des erfindungsgemäßen Verfahrens erlaubt insbesondere die Sterilisation einer Vielzahl von Behältnissen, wodurch die Sterilisationseinrichtung mit einer großen Anzahl an Behältnissen beschickt werden kann. Auf diese Weise kann der Durchsatz des Sterilisationsverfahrens gesteigert werden.

Gemäß einer bevorzugten Ausführungsform der vorliegenden Erfindung ist es vorgesehen, dass das mit einem medizinischen Befüllungsgut befüllte Behältnis in eine dicht verschlossene und/oder versiegelte Verpackung, insbesondere Bereitschaftspackung, eingebracht ist, wobei zumindest ein Teil der Verpackung wenigstens wasserdampfdurchlässig, insbesondere wasserdampf- und/oder gasdurchlässig, ausgebildet ist. In diesem Zusammenhang hat es sich als vorteilhaft erwiesen, wenn die Verpackung gleichermaßen sterilisiert wird. Eine solche Bereitschaftspackung, wie sie beispielsweise durch eine Blisterverpackung gebildet wird, besteht im Allgemeinen zumindest teilweise aus einem Sterilisationspapier, welches den Durchtritt von Wasserdampf und somit einen direkten Kontakt zwischen Wasserdampf und zu sterilisierendem Behältnis erlaubt.

Aus dem erfindungsgemäßen Verfahren resultiert somit ein mit einem medizinischen Befüllungsgut befülltes Behältnis, welches gemäß dem zuvor geschilderten Verfahren sterilisiert worden ist.

Das mit einem medizinischen Befüllungsgut befüllte Behältnis zeichnet sich im Vergleich zum Standard-Sattdampfverfahren und den Verfahren des Standes der Technik bei gleicher Sterilisationsdauer durch eine reduzierte Keimzahl bzw. durch Sterilität bei deutlich verkürzten Sterilisationszeiten aus.

Erfindungsgemäß ist das Behältnis ein medizinisches Behältnis, insbesondere eine Ampulle, eine Tube oder ein medizinisches Instrument, insbesondere ein Katheter oder eine Spritze, vorzugsweise eine Faltenbalg-Spritze oder eine Kolbenspritze, bevorzugt eine Kolbenspritze.

Gemäß einer bevorzugten Ausführungsform der vorliegenden Erfindung ist das Behältnis eine Kolbenspritze, insbesondere eine Einweg-Kolbenspritze, bevorzugt aus Kunststoff, vorzugsweise eine Kolbenspritze mit einem zylindrischen Hohlraum oder Grundkörper, welcher an einem Ende eine Öffnung zur Aufnahme eines Kolbens und an dem gegenüberliegenden Ende eine Düse aufweist.

In diesem Zusammenhang kann es vorgesehen sein, dass das mit einem medizinischen Befüllungsgut befüllte Behältnis eine mit einem Kathetergel befüllte Kolbenspritze, bevorzugt Einweg-Kolbenspritze, vorzugsweise aus Kunststoff, ist.

Gemäß einer besonders bevorzugten Ausführungsform der vorliegenden Erfindung ist das Behältnis eine Spritze, insbesondere Kolbenspritze, wobei die Spritze einen hohlen zylindrischen Grundkörper, welcher an einem Ende eine Öffnung zur Aufnahme eines Kolbens und an dem gegenüberliegenden Ende eine Düse aufweist, und einen parallel zur Erstreckungsrichtung des zylindrischen Grundkörpers in dem Hohlraum des zylindrischen Grundkörpers beweglichen Kolben umfasst, wobei der Hohlraum des zylindrischen Grundkörpers mit einem medizinischen Befüllungsgut, insbesondere mit einem flüssigen und/oder fließfähigen medizinischen Befüllungsgut, befüllt ist und gegebenenfalls die Düse verschlossen und/oder blockiert ist.

Dabei hat es sich im Rahmen der vorliegenden Erfindung als vorteilhaft erwiesen, wenn der Anteil bzw. das Volumen des Gases in dem Hohlraum der Spritze minimiert ist bzw. wenn die Spritze im Vakuum befüllt ist. Während der thermischen Sterilisation dehnt sich das Gas aus und erzeugt somit einen Druck auf die Innenwände des Behältnisses bzw. auf das Befüllungsgut. Das Behältnis, insbesondere die Spritze, wird daher vorteilhafterweise derart befüllt, dass die Ausdehnung bzw. das Volumen der bei der Befüllung im Hohlraum des Behältnisses, insbesondere der Spritze, verbleibenden Gas- und/oder Luftblasen minimiert ist, insbesondere vorzugsweise derart, dass keine Gas- und/oder Luftblasen im Hohlraum des Behältnisses, insbesondere der Spritze, verbleiben.

Das im Hohlraum der Spritzen verbleibende Gasvolumen kann beispielsweise durch eine optimierte Geometrie von Grundkörper und Kolben minimiert werden, so dass der Kolben den Hohlraum des Grundkörpers im nicht befüllten Zustand möglichst vollständig ausfüllt.

Im Rahmen der vorliegenden Erfindung hat es sich als vorteilhaft erwiesen, wenn die Befüllung der Spritze durch die Düse erfolgt ist, wobei zu Beginn der Befüllung, das von dem zylindrischen Grundkörper der Spritze und dem beweglichen Kolben eingeschlossene Volumen minimiert ist und während der Befüllung mit dem Befüllungsgut durch Bewegung des Kolbens entlang der Achse des Grundkörpers auf das gewünschte Maß expandiert wird. Auf diese Weise lässt sich eine besonders gleichmäßige und homogene Befüllung des Spritzenkörpers erreichen, welche darüber hinaus Ressourcen schonend ist. Das Befüllen der Spritze kann dabei durch Einpressen des Befüllungsguts in den Hohlraum der Spritze oder aber durch Einwirkung einer Zugkraft auf den Kolben der Spritze durchgeführt werden. Alternativ hierzu kann die Befüllung der Spritze aber auch über die der Düse der Spritze gegenüberliegende Kolbenöffnungsseite erfolgen, wobei diese Befüllung zur Vermeidung von Lufteinschlüssen unter reduziertem Druck erfolgen sollte.

Weiterhin kann die Düse der Spritze mit einem starren, insbesondere druckresistenten Verschluss versehen sein. Dies bedeutet, dass der Verschluss der Düse, gegebenenfalls mittels Unterstützung durch einen Stützdruck, einer Expansion des Befüllungsguts entgegenwirkt.

Alternativ kann es jedoch auch vorgesehen sein, dass die Düse der Spritze mit einem flexiblen Verschluss versehen ist. Ein derart flexibler Verschluss erlaubt eine Expansion des Befüllungsguts innerhalb gewisser Grenzen, so dass die Beaufschlagung mit einem Stützdruck deutlich geringer ausfallen kann als bei einem starren Verschluss der Düse. Des Weiteren kann bei einer besonderen Ausgestaltung des flexiblen Verschlusses ein Druckabbau gewährleistet sein, d. h. der flexible Verschluss erlaubt in dieser Ausgestaltung eine geringfügige Öffnung der Düse zum Druckabbau. Dies bedeutet beispielsweise, dass sich gegebenenfalls in dem Hohlraum der Spritze befindliches Restgasvolumen ausdehnen kann. Bei allen Ausführungsformen mit flexiblem Verschluss muss jedoch immer gewährleistet sein, dass das Befüllungsgut weder während der Sterilisation noch während der Lagerung aus dem Behältnis, insbesondere dem Spritzenkörper, entweicht. Des Weiteren darf ein solcher flexibler Verschluss auch nicht zu einem gesteigerten Rekontaminationsrisiko oder einer eingeschränkten Lagerfähigkeit des Behältnisses, beispielsweise aufgrund des Austritts von Feuchtigkeit bzw. Lösemitteln, führen.

Darüber hinaus kann eine Verpackung, insbesondere eine nach dem zuvor beschriebenen Verfahren sterilisierte Verpackung, ein mit einem medizinischem Befüllungsgut befülltes sterilisiertes Behältnis, insbesondere eine Spritze, enthalten, welches mit dem erfindungsgemäßen Verfahren sterilisiert wurde.

Die vorliegende Erfindung wird anhand der nachfolgenden Ausführungsbeispiele veranschaulicht, welche die vorliegende Erfindung jedoch keinesfalls beschränken sollen.

### Ausführungsbeispiele:

97 15-ml-Einwegkunststoffspritzen werden mit jeweils 11 ml eines Kathetergleitgels unter nicht-sterilen Bedingungen befüllt, wobei 10 zufällig ausgewählte Proben mit jeweils mindestens 10⁶ Sporen von *Geobacillus stearothermophilus* kontaminiert werden. Die Düsen der Spritze werden jeweils mit einer Verschlusskappe verschlossen, und von weiteren 20 Spritzen, welche zufällig ausgewählt sind, wird die tatsächliche Keimbelastung vor Verfahrensdurchführung bestimmt, wobei sich für alle 20 Systeme ein Befall mit Mikroorganismen nachweisen lässt.

Die Spritzen werden anschließend in Blisterverpackungen eingeschweißt, deren Rückseiten aus einem Wasserdampf durchlässigen Sterilisationspapier bestehen.

Drei weitere Spritzen, welche mit dem Kathetergleitgel gefüllt sind, werden mit einem Thermofühler ausgestattet, welcher fortlaufend die Temperatur des Kathetergleitgels misst und die Daten an den zentralen Rechner zur Steuerung des Sterilisationsverfahrens weiterleitet. Auch diese drei Proben werden anschließend verblistert.

Die insgesamt 100 Proben werden nun in eine Sterilisationseinrichtung eingebracht und einem Sterilisationsverfahren unterzogen, welches aus einem Aufheizverfahrensschritt, einem Sterilisationsverfahrensschritt und einem Abkühlverfahrensschritt besteht:
Zu Beginn des Verfahrens wird die in der Sterilisationseinrichtung vorhandene Atmosphäre ausgetauscht und durch die Sterilisationsatmosphäre unter Erhitzung ersetzt, indem erst ein fraktioniertes subatmosphärisches Vakuum angelegt wird, d. h. die Sterilisationseinrichtung wird mehrmals bis zu einem Unterdruck von 1 bar (Relativdruck von -1 bar) evakuiert und der Druck anschließend durch Dampfstöße wieder auf etwa Umgebungsdruck gebracht. Nachfolgend wird durch alternierende Dampf- und Druckluftstöße die Sterilisationsatmosphäre, welche aus einem Wasserdampf/Luft-Gemisch besteht, erzeugt und auf eine Temperatur von 123 °C und einen Relativdruck von 2,3 bar gebracht.

Der Druck und die Temperatur der Atmosphäre in der Sterilisationseinrichtung werden während der gesamten Verfahrensdauer ermittelt. Gleichfalls wird auch die Temperatur des Sterilisiergutes anhand der drei mit einem Thermofühler ausgestatteten Referenzproben bestimmt.

Nach Abwarten der Ausgleichszeit beginnt der eigentliche Sterilisationsverfahrensschritt bei einer Temperatur der Referenzproben von 120 °C. Die Temperatur der Sterilisationsatmosphäre wird für 10 Minuten auf 123 °C gehalten, wobei der Relativdruck 2,3 bar beträgt.

Nach Ablauf der 10 Minuten wird der Druck in der Sterilisationseinrichtung durch einen einzelnen Druckluftstoß um 0,2 bis 0,5 bar erhöht, wodurch eine gesteigerte bzw. verstärkte Kondensation des Wasserdampfes und eine entsprechende gesteigerte Ausnutzung der im Wasserdampf befindlichen Energie eintritt.

Die Stufe des Sterilisationsverfahrensschritts unter erhöhtem Druck wird für insgesamt 3 Minuten aufrechterhalten, wobei die Temperatur in der Sterilisationsatmosphäre während dieser Zeit fallen darf, jedoch nicht die Temperatur im Sterilisationsgut. Der eigentliche Sterilisationsverfahrensschritt erstreckt sich somit über einen Zeitraum von 13 Minuten.

An den Sterilisationsverfahrensschritt schließt sich der mehrstufige Abkühlverfahrensschritt an. Zuerst wird in einem dreistufigen Prozess die Temperatur der Atmosphäre der Sterilisationseinrichtung auf ca. 40 °C gekühlt, wobei die ganze Zeit der Druck in der Sterilisationseinrichtung durch einen Stützdruck konstant gehalten wird und ca. 2,6 bar (relativ) beträgt. Die Temperatur des Sterilisierguts sinkt in dieser Zeit auf ca. 80 °C. An diesen Abkühlvorgang schließt sich ein mehrstufiger Trocknungsprozess an, bei welchem mehrmals die Temperatur der Atmosphäre in der Sterilisationseinrichtung erhöht und gleichzeitig der Druck in der Sterilisationseinrichtung erniedrigt wird, um das kondensierte Wasser wieder in die Gasphase zu überführen. Anschließend wird der Druck wieder gesteigert, um eine Expansion des Kathetergleitgels in den Spritzen entgegenzuwirken.

Dieser Vorgang wird so oft wiederholt, bis kein kondensiertes Wasser mehr in der Sterilisationseinrichtung vorhanden ist.

Nachfolgend wird der Druck innerhalb der Sterilisationseinrichtung nochmals mittels Druckluft auf ca. 2 bar relativ eingestellt, die Sterilisationseinrichtung entlüftet und geöffnet.

Blisterverpackungen und Spritzen werden anschließend auf mechanische Beschädigung oder ein Auslaufen des Kathetergleitgels untersucht. Alle Proben befinden sich jedoch in einwandfreiem Zustand.

Nachfolgend werden die 97 verblisterten Spritzen unter sterilen Bedingungen ausgepackt und sowohl die Spritzen als auch das Kathetergleitgel und die Blisterverpackungen auf Mikroorganismen untersucht. In keiner der Proben kann eine Kontamination mit Mikroorganismen nachgewiesen werden, auch nicht in den 10 ausgewählten Proben, welche mit den Sporen des *Geobacillus stearothermophilus* versetzt wurden.

Durch weitere Versuche wird bestimmt, dass der 13-minütige Sterilisationsverfahrensschritt des zuvor geschilderten Verfahrens einer 18-minütigen Sterilisation unter Standardbedingungen entspricht, d. h. das durchgeführte erfindungsgemäße Verfahren besitzt einen F₀-Wert von 18.

Zum Vergleich werden dem erfindungsgemäßen Verfahren analoge Versuche durchgeführt, bei denen lediglich die zusätzliche Druckbeaufschlagung während der Sterilisationsphase weggelassen wird. Für das in der Art durchgeführte Sterilisationsverfahren kann ein F₀-Wert von 15,5 ermittelt werden. Darüber hinaus können in zwei der mit den Sporen von *Geobacillus stearothermophilus* kontaminierten Proben noch vermehrungsfähige Sporen nachgewiesen werden.

Das zuvor geschilderte erfindungsgemäße Verfahren sowie das Vergleichsverfahren sind in der nachfolgenden Tabelle 1 dem Standard-Sattdampfverfahren gegenübergestellt.

**Tabelle 1:**

| | **Standard-Sattdampf (nichterfindungsgemäß)** | **Wasserdampf/LuftGemisch (nichterfindungsgemäß)** | **Wasserdampf/LuftGemisch und zusätzliche Druckbeaufschlagung (erfindungsgemäß)** |
|---|---|---|---|
| Temperatur [°C] | 121 | 123 | 123 |
| Relativdruck [bar] | 1 | 2,3 | 2,3 (2,6)² |
| Zeit | 15 | 13 | 13 |
| F₀-Wert | 15¹ | 15,5 | 18 |

| | | | |
|---|---|---|---|
| ¹: **per** Definition ²: nach Druckbeaufschlagung in zweiter Stufe | | | |

Es zeigt sich, dass mit dem erfindungsgemäßen Verfahren in kürzerer Zeit eine deutlich bessere Keimreduktion erreicht werden kann. Das erfindungsgemäße Verfahren ermöglicht somit in einfacher und effizienter Weise eine verbesserte Ausnutzung der im Wasserdampf enthaltenen Energie.

## Patentansprüche

1. Verfahren zur thermischen Sterilisation eines mit einem flüssigen oder fließfähigen medizinischen Befüllungsgut befüllten medizinischen Behältnisses, wobei das Verfahren einen Sterilisationsverfahrensschritt umfasst, bei dem eine thermische Sterilisation eines mit einem medizinischen Befüllungsgut befüllten Behältnisses in Gegenwart einer Wasserdampf enthaltenden Sterilisationsatmosphäre bei Temperaturen von mindestens 100 °C und unter erhöhtem Druck durchgeführt wird,
wobei dem Sterilisationsverfahrensschritt ein Aufheizverfahrensschritt vorgeschaltet ist;
wobei der Sterilisationsverfahrensschritt in einem Absolutdruckbereich von 1,05 bis 11 bar durchgeführt wird; wobei der Sterilisationsverfahrensschritt mindestens eine erste Verfahrensstufe und eine zweite, der ersten Verfahrensstufe nachfolgende Verfahrensstufe umfasst, wobei erste und zweite Verfahrensstufe unter voneinander verschiedenen Drücken durchgeführt werden, wobei der Druck in der zweiten Verfahrensstufe gegenüber dem Druck in der ersten Verfahrensstufe erhöht wird, wobei die relative Druckerhöhung von der ersten zu der zweiten Verfahrensstufe des Sterilisationsverfahrensschritts mindestens 0,01 bar beträgt und wobei die Druckerhöhung von der ersten zu der zweiten Verfahrensstufe des Sterilisationsverfahrensschritts mittels Eintrag von Luft erfolgt; und
wobei dem Sterilisationsverfahrensschritt ein Abkühlverfahrensschritt nachgeschaltet ist, wobei während des Abkühlverfahrensschritts zumindest vorübergehend ein Stützdruck angewendet wird derart, dass dem in dem mit einem medizinischen Befüllungsgut befüllten medizinischen Behältnis vorhandenen Druck entgegengewirkt wird.

2. Verfahren nach Anspruch 1 **dadurch gekennzeichnet, dass** der Sterilisationsverfahrensschritt in Gegenwart eines Wasserdampf/Gas-Gemisches durchgeführt wird.

3. Verfahren nach Anspruch 1 oder 2, **dadurch gekennzeichnet, dass** das Verfahren in einer geschlossenen Sterilisationseinrichtung, insbesondere in einem gasdicht verschlossenen Druckbehälter, vorzugsweise in einer Autoklaviereinrichtung, durchgeführt wird.

4. Verfahren nach einem der vorangehenden Ansprüche, **dadurch gekennzeichnet, dass** die Sterilisationsatmosphäre durch Injektion von Wasserdampf/Druckluft-Gemischen in die Sterilisationseinrichtung erzeugt wird.

5. Verfahren nach einem der vorangehenden Ansprüche, **dadurch gekennzeichnet, dass** der Wasserdampf aus destilliertem und/oder vollentsalztem Wasser erzeugt wird, und dass die Druckluft aus steriler Luft erzeugt wird.

6. Verfahren nach einem der vorangehenden Ansprüche, **dadurch gekennzeichnet, dass** der Sterilisationsverfahrensschritt in einem Temperaturbereich zwischen 100 und 180 °C durchgeführt wird.

7. Verfahren nach einem der vorangehenden Ansprüche, **dadurch gekennzeichnet, dass** der Sterilisationsverfahrensschritt in einem Absolutdruckbereich von 1,1 bis 5 bar durchgeführt wird.

8. Verfahren nach einem der vorangehenden Ansprüche, **dadurch gekennzeichnet, dass** der Sterilisationsverfahrensschritt 2 bis 10 Verfahrensstufen umfasst, wobei mindestens 2 Verfahrensstufen bei unterschiedlichen Drücken durchgeführt werden.

9. Verfahren nach einem der vorangehenden Ansprüche, **dadurch gekennzeichnet, dass** das Verfahren zumindest während des Sterilisationsverfahrensschritts über die Messung und Regelung von Druck und Temperatur tens einer in der Sterilisationseinrichtung befindlichen Referenzprobe gesteuert und/oder kontrolliert wird.

10. Verfahren nach einem der vorangehenden Ansprüche, **dadurch gekennzeichnet, dass** zumindest während des Sterilisationsverfahrensschritts Druck und Temperatur bestimmt und eingestellt werden

11. Verfahren nach Anspruch 9 oder 10, **dadurch gekennzeichnet, dass** die Druck- und Temperaturdaten zur Kontrolle und Steuerung zumindest des Sterilisationsverfahrensschritts verwendet werden.

12. Verfahren nach einem der vorangehenden Ansprüche, **dadurch gekennzeichnet, dass** während des Aufheizverfahrensschritts Druck und Temperatur der Atmosphäre innerhalb der Sterilisationseinrichtung geändert werden.

13. Verfahren nach einem der vorangehenden Ansprüche, **dadurch gekennzeichnet, dass** während des Abkühlverfahrensschritts Druck und Temperatur der Atmosphäre innerhalb der Sterilisationseinrichtung geändert werden.

14. Verfahren nach einem der vorangehenden Ansprüche, **dadurch gekennzeichnet, dass** das medizinische Befüllungsgut ein Arzneimittel, Pharmazeutikum oder Medizinprodukt, insbesondere ein Gleitgel, vorzugsweise Kathetergleitgel, ist.

15. Verfahren nach einem der vorangehenden Ansprüche, **dadurch gekennzeichnet, dass** das Behältnis eine Ampulle, eine Tube oder ein medizinisches Instrument, insbesondere ein Katheter oder eine Spritze, vorzugsweise eine Faltenbalg-Spritze oder eine Kolbenspritze, bevorzugt eine Kolbenspritze, ist.

## Claims

1. A method for thermal sterilization of a medical container, filled with a liquid or flowable medical filling product, the method comprising a sterilization process step in which a thermal sterilization of a container filled with a medical filling product is performed in the presence of a sterilization atmosphere containing water vapor at temperatures of at least 100°C and at an increased pressure,
wherein a heating process step is connected upstream of the sterilization process step; wherein the sterilization process step is carried out in an absolute pressure range of 1.05 to 11 bars;
wherein the sterilization process step comprises at least a first process step and a second process step subsequent to the first process step, wherein the first and second process steps are carried out at pressures differing from each other, wherein the pressure in the second process step is increased as opposed to the pressure in the first process step, wherein the relative pressure increase from the first to the second process step of the sterilization process step is at least 0.01 bar, and wherein the pressure increase from the first to the second process step of the sterilization process step is carried out by means of introducing air; and
wherein a cooling process step is connected downstream of the sterilization process step, wherein during the cooling process step a support pressure is used, at least temporarily, such that the pressure present in the medical container filled with a medical filling product is counteracted.

2. The method according to claim 1, **characterized in that** the sterilization process step is carried out in the presence of a water vapor/gas mixture.

3. The method according to claims 1 or 2, **characterized in that** the method is carried out in a closed sterilization unit, in particular in a pressure vessel that is closed in an airtight manner, preferably in an autoclave unit.

4. The method according to one of the previous claims, **characterized in that** the sterilization atmosphere is created by means of injection of water vapor/pressurized air mixtures into the sterilization unit.

5. The method according to one of the previous claims, **characterized in that** the water vapor is created from distilled and/or fully desalinated water, and that the pressurized air is created from sterile air.

6. The method according to one of the previous claims, **characterized in that** the sterilization process step is carried out in a temperature range of between 100 and 180°C.

7. The method according to one of the previous claims, **characterized in that** the sterilization process step is carried out in an absolute pressure range of 1.1 to 5 bars.

8. The method according to one of the previous claims, **characterized in that** the sterilization process step comprises 2 to 10 process phases, wherein at least 2 process phases are carried out at different pressures.

9. The method according to one of the previous claims, **characterized in that** the method may be regulated and/or controlled via the measurement and regulation of pressure and the temperature of a reference sample present in the sterilization unit, at least during the sterilization process step.

10. The method according to one of the previous claims, **characterized in that** the pressure and temperature are determined and adjusted, at least during the sterilization process step.

11. The method according to claims 9 or 10, **characterized in that** the pressure and temperature data is utilized for controlling and regulating at least the sterilization process step.

12. The method according to one of the previous claims, **characterized in that** the pressure and temperature of the atmosphere within the sterilization unit are modified during the heating process step.

13. The method according to one of the previous claims, **characterized in that** the pressure and temperature of the atmosphere within the sterilization unit are modified during the cooling process step.

14. The method according to one of the previous claims, **characterized in that** the medical filling product is a drug, pharmaceutical, or a medical product, in particular a lubricant, preferably a catheter lubricant.

15. The method according to one of the previous claims, **characterized in that** the container is an ampule, a tube, or a medical instrument, in particular a catheter or a syringe, preferably a corrugated bellows syringe, or a piston-type syringe, preferably a piston-type syringe.

## Revendications

1. Procédé de stérilisation thermique d'un récipient à usage médical rempli d'un produit de remplissage à usage médical, liquide ou fluide, le procédé comprenant une opération de stérilisation, dans laquelle une stérilisation thermique d'un récipient rempli d'un produit de remplissage à usage médical est mise en oeuvre en présence d'une atmosphère de stérilisation contenant de la vapeur d'eau à des températures d'au moins 100°C et sous une pression élevée,
l'opération de stérilisation étant précédée d'une opération de montée en température,
l'opération de stérilisation étant mise en oeuvre sous une pression absolue comprise dans la plage de 1,05 à 11 bar,
l'opération de stérilisation comprenant au moins une première étape et une deuxième étape suivant la première étape, la première et la deuxième étapes étant mises en oeuvre sous des pressions différentes l'une de l'autre, la pression dans la deuxième étape étant augmentée par rapport à la pression dans la première étape, l'augmentation de la pression relative de la première étape à la deuxième étape de l'opération de stérilisation étant d'au moins 0,01 bar, et l'augmentation de la pression de la première à la deuxième étape de l'opération de stérilisation s'effectuant grâce à un apport d'air ; et
l'opération de stérilisation étant suivie d'une opération de refroidissement, une pression d'appui étant, au moins provisoirement, et pendant l'opération de refroidissement, mise en oeuvre de façon à contrecarrer la pression présente dans un récipient à usage médical rempli d'un produit de remplissage à usage médical.

2. Procédé selon la revendication 1, **caractérisé en ce que** l'opération de stérilisation est mise en oeuvre en présence d'un mélange vapeur d'eau/gaz.

3. Procédé selon la revendication 1 ou 2, **caractérisé en ce que** le procédé est mis en oeuvre dans un dispositif fermé de stérilisation, en particulier dans un récipient sous pression obturé d'une manière étanche aux gaz, de préférence dans un dispositif d'autoclavage.

4. Procédé selon l'une des revendications précédentes, **caractérisé en ce que** l'atmosphère de stérilisation est produite par injection de mélanges vapeur d'eau/air comprimé dans le dispositif de stérilisation.

5. Procédé selon l'une des revendications précédentes, **caractérisé en ce que** la vapeur d'eau est produite à partir d'eau distillée et/ou entièrement déminéralisée, et que l'air comprimé est produit à partir d'air stérile.

6. Procédé selon l'une des revendications précédentes, **caractérisé en ce que** l'opération de stérilisation est mise en oeuvre sur une plage de températures entre 100 et 180°C.

7. Procédé selon l'une des revendications précédentes, **caractérisé en ce que** l'opération de stérilisation est mise en oeuvre sous une pression absolue comprise dans la plage de 1,1 à 5 bar.

8. Procédé selon l'une des revendications précédentes, **caractérisé en ce que** l'opération de stérilisation comprend 2 à 10 étapes, au moins 2 étapes étant mises en oeuvre sous des pressions différentes.

9. Procédé selon l'une des revendications précédentes, **caractérisé en ce que** le procédé est piloté et/ou commandé au moins pendant l'opération de stérilisation, par l'intermédiaire de la mesure et de la régulation de la pression et de la température d'au moins un échantillon de référence se trouvant dans le dispositif de stérilisation.

10. Procédé selon l'une des revendications précédentes, **caractérisé en ce que** la pression et la température sont déterminées et ajustées au moins pendant l'opération de stérilisation.

11. Procédé selon la revendication 9 ou 10, **caractérisé en ce que** les données relatives à la pression et à la température sont utilisées pour la commande et le pilotage d'au moins l'opération de stérilisation.

12. Procédé selon l'une des revendications précédentes, **caractérisé en ce que** la pression et la température de l'atmosphère sont modifiées à l'intérieur du dispositif de stérilisation pendant l'opération de montée en température.

13. Procédé selon l'une des revendications précédentes, **caractérisé en ce que** la pression et la température de l'atmosphère à l'intérieur du dispositif de stérilisation sont modifiées pendant l'opération de refroidissement.

14. Procédé selon l'une des revendications précédentes, **caractérisé en ce que** le produit de remplissage à usage médical est un médicament, un produit pharmaceutique ou un produit à usage médical, en particulier un gel lubrifiant, de préférence un gel lubrifiant pour cathéter.

15. Procédé selon l'une des revendications précédentes, **caractérisé en ce que** le récipient est une ampoule, un tube ou un instrument médical, en particulier un cathéter ou une seringue, de préférence une seringue en accordéon ou une seringue à piston, de préférence une seringue à piston.
